# EUROPEAN PATENT SPECIFICATION

(11) **EP 1 587 477 B1**
(45) Date of publication and mention of the grant of the patent: **26.10.2011**
(21) Application number: 04700231.6
(22) Date of filing: 05.01.2004
(51) Int. Cl.: A61K 31/185, A61K 45/06, A61K 31/255, A61K 31/095, A61Q 11/00, A61Q 19/00, A61Q 19/10, A61K 8/46, C07C 303/32, C07C 309/42, C07C 303/06, A61K 9/00, A61P 1/02, A61P 17/00, A61P 17/02, A61P 43/00

(54) **ODORLESS FORMULATION FOR TREATING MUCOSAL DISCONTINUITIES**
GERUCHLOSE FORMULIERUNG ZUR BEHANDLUNG VON SCHLEIMHAUTDISKONTINUITÄTEN
FORMULATION NON ODORANTE POUR LE TRAITEMENT DES DISCONTINUITES DES MUQUEUSES

(30) Priority: 03.01.2003 US 437777 P; 21.05.2003 US 443446; 21.05.2003 US 443445
(43) Date of publication of application: 26.10.2005
(73) Proprietor: EPIEN Medical, Inc., St. Paul, MN 55110 (US)
(72) Inventor: BASARA, Michael, Hugo, MN 55038 (US)
(74) Representative: Smyth, Gyles Darren
(86) International application number: PCT/US2004/000133
(87) International publication number: WO 2004/062580

(56) References cited:
- EP-A- 1 145 707
- WO-A-00/00166
- WO-A1-00/07540
- GB-A- 820 659
- US-A- 4 082 841
- US-A- 4 331 653
- US-A1- 2003 185 768
- HIROTSUGU YOSHIDA ET AL.: "Effect of sodium guaiazulene-3-sulfonate on experimental stomatitis induced by acetic acid in hamster cheek pouch" JAPANESE PHARMACOLOGY AND THERAPEUTICS, vol. 14, no. 3, 1896, pages 217-224, XP008094785
- BUDAVARI ET AL: 'THE MERK INDEX.' 1989, page 7212, XP008056122
- RHODUS L ET AL: "An Evaluation of a Chemical Cautery Agent and an Anti-Flammatory Oinment for the Treatment of Recurrent Aphthous Stomatitis: A Pilot Study", QUINTESSENZ, QUINTESSENZ VERLAG, BERLIN, DE, vol. 29, no. 12, 1 January 1998 (1998-01-01), pages 769-773, XP007908002, ISSN: 0033-6580

## Description

The present invention relates to odorless formulations suitable for use in treating mucosal discontinuities. The present invention also includes a method for making the formulations and disclosed is a method for using the formulations. The formulations may be used in a method for exfoliating skin, including facial skin, skin on the bottom of the feet and elbows.

The term, "mucosal discontinuities" as used herein refers to discontinuities which are present or which are inflicted on mucosal tissue of living beings. Mucosal discontinuities include wounds which are internal or external bodily injuries or lesions which are caused by a physical force or by another mechanism. The physical force is one or more of a mechanical, chemical, viral, bacterial, or thermally induced physical force. The physical force disrupts the normal continuities of biologic structures of living beings.

Mucosal discontinuities include contusions, wounds in which the skin is unbroken, incision, wounds in which the skin is broken by a cutting instrument, lacerations, and wounds in which the skin is broken by a dull or blunt instrument. Discontinuities include wounds caused by accident or by surgical procedures.

Patients who suffer major wounds and other discontinuities benefit from treatment that enhances healing and pain relief. Wound healing mechanisms 5 comprise a series of processes whereby tissue, such as mucosal tissue, is repaired, In particular, in repair, specialized tissue is regenerated. New tissue is reorganized.

Would healing typically comprises three phases. A first phase is an inflammation phase that lasts up to about 3 days. A seconds phase is a cellular proliferation phase that lasts from about 3 to 12 days. A third phase is a remodeling phase that lasts from about 3 days to 6 months.

During the first inflammation phase, platelet aggregation and clotting form a matrix which traps plasma proteins and blood cells to induce the influx of various type of cells. During the second cellular proliferation phase, new connective or granulation tissue and blood vessels are formed. During the third remodeling phase, granulation tissue is replaced by a network of collagen and elastin fibers leading to the formation of scar tissue.

When cells are injured or killed as a result of a wound, the wound healing step is desirable to resuscitate the injured cells and to produce new cells to replace the dead cells. The healing process produces a reversal of cytotoxicity, a suppression of inflammation, and a stimulation of cellular viability and proliferation. Wounds typically require low levels of oxygen in the initial stages of healing to suppress oxidative damage and higher levels of oxygen in the later stages of healing to promote collagen formation by fibroblasts.

One type of mucosal discontinuity includes aphthous ulcers. Aphthous ulcers are believed to be caused by a virus, in some instances, as well as genetic, trauma, hormonal changes, and gastrointestinal factors.

Aphthous ulcers have shapes that range from single, multiple, round, to oval shaped. The ulcers range in size from 2-40 mm. The ulcers occur on mucus membranes of the tongue, cheeks, lips, soft and hard pallets, gingiva, pharynx and on the floor of the mouth. The ulcers are also found in the genital, anal, and in conjunctival mucosae.

Aphthous ulcers are extremely painful lesions. The ulcers appear as small macular red lesions. The ulcerated area quickly undergoes necrosis, leaving a sharply defined rounded ulcer, varying from about 2 to 5 mm in diameter. The ulceration is fairly deep with a yellow white base representing the tissue at the surface. The margin of the ulcer somewhat indurated and the margin of the mucosae has a surrounding erythematous zone. The marginal erythema ranges from slight to extensive, depending upon the degree of the secondary bacterial involvement.

The aphthous ulcer is present for about seven days and it undergoes gradual healing. It heals as a general rule in approximately 10 - 14 days and does not tend to leave a scar. Characteristically, there is a recurrent pattern of one of more of these ulcers. The ulcers recur as soon as one month apart and there are cases where, for a period of years, the individual is never without ulcers. New ulcers form as the existing lesions heal. In other cases, aphthous ulcer attacks may occur two to three times during a year. The lesions also often appear following some intense emotional stress, but they may first appear following a gradual change in environment or following an emotional situation in a non-familiar environment.

Aphthous ulcers have been found to occur in greater frequency in women. The ulcers appear several days prior to the menstrual period. The first encounter with aphthous stomatitis for women frequently follows the onset of menstruation. Women susceptible to these lesions often report freedom from the lesions during pregnancy. There is a tendency for a greater frequency of these lesions in females than in male. Although they occur at any age level, the ulcers occur more often in adults.

The term "Periadenitis Mucosa Necrotica Recurrens" is sometimes used to describe aphthae that coalesce to form an elongated, deep ulcerated area. From a symptomatic standpoint, it has found that about 24 - 48 hrs. before onset of an aphthous lesion, there is a vague discomfort, sometimes described as a tingling sensation in the area. As the tissue undergoes necrosis and an ulcer forms, the lesions become very painful. The aphthous lesions are often considered the most painful oral ulcerations. The discomfort may become particularly intense during periods of fatigue.

The histopathology of the disease is one where the microscopy picture is non-specific, generally showing an ulceration of the mucosae. The surface epithelium exhibits a central area of destruction. The connective tissue is densely infiltrated with lymphocytes, polymorphonuclear leukocytes, plasma cells, and histocytes. There is evidence of active fibrosis at the base and sides of the ulcerated area.

Differential diagnosis of aphthous ulcers includes traumatic ulcers, acute herpetic stomatitis, stomatitis medicamentosa, and erythema multiforme. The diagnosis of aphthous stomatitis is based upon the clinical manifestation and the patient's history. Biopsies are usually unnecessary due the extreme discomfort involved and are avoided unless necessary to rule out other lesions considered differentially diagnostic.

Many substances in agents have been used in an attempt to cure and or relieve the discomfort of aphthous lesion. For example, cauterizing drugs, such as phenol, chromic acid, alum and silver nitrate, have been used for many years. These agents alleviate pain by destruction of small nerve endings. The healing time of the lesion is prolonged due to the escharotic action of these drugs on the surface epithelium and the active fibrosis at the base and sides of the ulcerated areas. Vitamins have also been tried with inconsistent results. Antibiotics have been used with conflicting results.

One observer found that Aureomycin applied locally, three times a day appeared to have a definite effect. With treatment, the duration of the ulcers was reduced from about 10-5 days and there was an analgesic effect lasting one half to two hours. Temporary relief has also been sought and sometimes achieved by using milk of magnesia or heavy syrups. Other more exotic remedies have been tried with little or no success. These remedies include vaccination with cowpox virus, and nutrient supplements.

U.S. Patent Nos. 3,920,835, 3,984,556, and 3,988,470, all issued to Van Scott et al., disclose methods for treating acne, dandruff and palmar keratosis, respectively. The methods generally comprise applying to an affected area a topical composition that comprises about 1% to 20% of a lower aliphatic compounds composition that contains from 2 to 6 carbon atoms selected from a group consisting of alpha hydroxy acid, alpha-ketoacids and esters thereof, and 3-hydroxybutyric acid in a pharmaceutically acceptable carrier.

U.S. Patent No. 4,416,982 issued to Tauda et al. discloses a method for decomposing hydrogen peroxide by reacting the hydrogen peroxide with a phenol or aniline derivative in the presence of peroxidase.

US 4,331,653 discloses styptic compositions which may contain a metal phenolsulfonate selt.

Rhodus et al., "An Evaluation of a Chemical Cautery Agent and an Anti-inflammatory Ointment for the Treatment of Recurrent Aphthous Stomatitis: A Pilot Study", Quintessence International, 1998, Vol. 29, Nr. 12, pages 769-773 discloses then known Cautery agent Debacterol [RTM], which is a complicated mixture derived from a wood distillate. It has a strong odour and a strong bitter and acidic taste.

One prior art formulation is manufactured by Northern Research Laboratories of St. Paul, Minnesota. The formulation is a reddish-brown material that has a strong phenolic odor.

### Description of the Drawings

Figure 1a is a schematic view of a prior art formulations that includes sulfuric acid, Beechwood creosote and purified water.
Figure 1b is a schematic view of the formulation of the present invention is and its uses in treating conditions of skin and the oral cavity.
Figure 2 is a formula view of chemicals in the formulation of the present invention.
Figure 3 is a perspective view of devices for application of the formulation of the present invention.

### Summary

One embodiment of the present invention includes a method for making a formulation suitable for treating mucosal discontinuities, comprising:
providing phenol, guaiacol, sulfuric acid and, optionally, water in preselected concentrations;
reacting the guaiacol and phenol with the sulfuric acid and, optionally, water to make phenolsulfonic acid and guaiacolsulfonic acid and, optionally, combining with free water and free acid to produce a formulation having a preselected concentration of preselected isomers of phenolsulfonic acid, preselected isomers of guaiacolsulfonic acid, and optionally, free acid and free water.

Another embodiment of the present invention includes a formulation suitable for use in the treatment of mucosal discontinuities, comprising: isomers of phenolsulfonic acid, isomers of guaiacolsulfonic acid, and optionally, free acid and free water, the formulation being obtainable by the above mentioned method.

Another embodiment of the present invention includes a formulation suitable for use in the treatment of mucosal discontinuities, comprising: phenolsulfonic acid and isomers of phenolsulfonic acid; guaiacolsulfonic acid and isomers of guaiacolsulfonic acid and mono- and bis-forms of guaiacolsulfonic acid: ammonium phenolsulfonate; and potassium guaiacolsulfonate, the isomers of phenolsulfonic acid and of guaiacolsulfonic acid being obtainable by the above mentioned method.

Another embodiment of the present invention includes a formulation suitable for use in the treatment of oral mucosal discontinuities, comprising: phenolsulfonic acid in a concentration of 25-80% by weight; guaiacolsulfonic acid in a concentration of 25-80% by weight; free sulfuric acid in a concentration of 0 to 32% by weight; and water in a concentration of 0 to 3% by weight.

One other embodiment includes a formulation suitable for use in the treatment of skin mucosal discontinuities, comprising: phenolsulfonic add in a concentration of 25-80% by weight; ammonium phenolsulfonate in a concentration of 0 to 5% by weight; guaiacolsulfonic acid in a concentration of 25-80% by weight; free sulfuric acid in a concentration of 0 to 32% by weight: and water in a concentration of 0 to 3% by weight.

Another embodiment of the present invention includes a kit suitable for use in the treatment of mucosal discontinuities comprising the above mentioned formulation, a container for containing the formulations and a mechanism for delivering the formulation to a mucosal discontinuity.

Another embodiment includes a device suitable for use in the treatment of mucosal discontinuities, comprising: a syringe, and the formulation of the present invention contained in the syringe.

One other embodiment of the present invention includes a system comprising: ingredients that include phenolsulfonic acid, guaiacolsulfonic acid, ammonium phenolsulfonate, potassium guaiacolsulfonate, water and free acid; and a mechanism calibrated to mix preselected amounts of two or more of the ingredients together to make a formulation suitable for use in the treatment of a specific type of mucosal discontinuity.

One other embodiment includes an exfoliating composition. The exfoliating composition includes a mixture comprising phenolsulfonic acid, guaiacolsulfonic acid and, optionally, sulfosalicylic acid.

Preferred features of the above embodiments are set out below, and in the dependent claims.

### Detailed Description

In its method and system aspects, the present invention includes a method and system for making formulations which can be tailored for treating specific types of mucosal discontinuities. The method includes providing phenol, guaiacol, sulphuric acid and; optionally, water, in preselected concentrations, and reacting the guaiacol and phenol with the sulphuric acid and, optionally, water to make phenolsulfonic acid and guaiacolsulfonic acid and, optionally, combining with free water and free acid. The preselected concentrations of guaiacol, phenol, sulphuric acid, free acid and water produce a formulation having a preselected concentration of four isomers of phenolsulfonic acid, seven isomers of guaiacolsulfonic acid, and for some embodiments, free acid and free water. The specific isomer concentrations and concentration ratios are preselected by selecting specific reaction parameters such as time, temperature, and concentration of reactants that produce the preselected isomer concentration profile.

In another embodiment, the phenolsulfonic acid is further treated with ammonium hydroxide to make ammonium phenolsuffonate. The gaiacolsulfonic acid is treated with potassium or zinc hydroxide to make potassium guaiacolsulfonate or zinc guaiacolsulfonate.

The system of the present invention includes the ingredients phenolsulfonic acid, guaiacolsulfonic acid, optionally sulfosalicylic acid, ammonium phenolsulfonate, potassium guaiacolsulfonate, water and sulfuric acid which is National Formulary (NF). The system also includes a mechanism calibrated to mix preselected amounts of the ingredients together to make a formulation for use in treating a specific type of mucosal discontinuity.

Formulations according to the present invention may be used in a method for exfollating skin. The method comprises exposing skin, which has been cleansed, to a solution or a cream or lotion or paste or gal or foam comprising one or more of sulphonated phenolic compounds including phenolsulfonic acid, guaiacolsulfonic acid, and sulfosalicylic acid, for a time effective to warm the skin and produce a tingling sensation. The solution or cream or paste or gel or foam comprises a mixture of phenolsulfonic acid and one or more of guaiacolsulfonic acid and sulfosalicylic acid. Some formulations optionally include free acid. In one embodiment the mixture includes:

| Component | Concentration Ranges |
|---|---|
| Phenolsulfonic Acid | 25-80% by weight |
| Guaiacolsulfonic Acid | 25-80% by weight |
| Ammonium Phenolsulfonate | 0-5% by weight |
| Free Sulfuric Acid | 0-3% by weight |
| Water | 13-30% by weight |
| Colorant | 0.075-0.020% by weight |

One exfoliating composition of the present invention includes two isomers of phenolsulfonic acid and four isomers of guauacolsulfonic acid. Water is added as a diluent in this formulation. Another exfoliating composition of the present invention additionally includes sulfosalicylic acid. One other exfoliant composition embodiment includes citric acid. An embodiment used as a foot exfoliant includes ammonium phenolsulfonate and zinc phenolsulfonate. These embodiments include water or alcohol or a mixture of water and alcohol as a diluent.

It is believed that the exfoliating compositions (exfoliant) of the present invention macerate the stratus corneum without penetrating into the underlying skin tissue. The exfoliant produces a precipitation reaction that cleanses the skin, particularly lesions on the surface of the skin, without damaging underlying layers. The cleansing aids in repair of the lesions and In the growth of new skin.

One specific embodiment of the solution has the following concentration ranges:

| **Component** | **Concentration Ranges** |
|---|---|
| Phenolsulfonic Acid | 25-80% by weight |
| Guaiacolsulfonic Acid | 25-80% by weight |
| Ammonium Phenolsulfonate | 0-32% by weight |
| Free Sulfuric Acid | 0-32% by weight |
| Water | 13-30% by weight |
| Colorant | 0.000-0.075% by weight |

Another formulation embodiment is as follows:

| **Component** | **Concentration Ranges** |
|---|---|
| Phenolsulfonic Acid | 25-80% by weight |
| Guaiacolsulfonic Acid | 25-80% by weight |
| Ammonium Phenolsulfonate | 0-5% by weight |
| Free Sulfuric Acid | 0-3% by weight |
| Water | 13-30% by weight |
| Colorant | 0.000-0.075% by weight |

As used herein, the term "mucosal discontinuity" refers to lesions, ulceration, or inflammation on the moist linings of the buccal cavity, nasal cavity, gastrointestinal tract, respiratory tract, conjunctiva, vagina, colon, urinary bladder, and urethra.

As used herein, "Exfoliation" refers to a detachment and shedding of superficial cells of an epithelium or from any tissue surface. Tissue surfaces include but are not limited to facial skin, skin on the soles of feet, knees, elbows, legs, arms, and other skin areas. Exfoliation also includes detachment or shedding of calloused skin, such as skin on the sole and heel of a foot, skin on an elbow and callouses on other parts of the body.

"Pharmaceutical" refers to a formulation administered to the skin which renders a benefit or an effect for treating or preventing an abnormal biological condition or a disease.

"Skin Atrophy" refers to a thinning and/or a general degradation of the dermis layer of mammalian skin, often characterized by a degree in collagen and/or elastic, as well as a doubling of fibroblast cells. Skin atrophy is a natural result of the aging process, but may be caused by either intrinsic or extrinsic factors such as photo damage, burns or chemical damage, or by exposure to pollutants or allergens such as cigarette smoke. Skin atrophy is often an undesirable side-effect resulting from treatment with alpha hydroxy carboxylic acids.

In one example, a formulation suitable for treating gingivitis, one type of mucosal discontinuity, includes sulfonic acid and little or, no free acid. In another example, phenolsulfonic acids and guaiacolsulfonic acids in a formulation with free sulfuric acid are suitable for use to treat periodontal disease, another type of mucosal discontinuity. In general, a formulation that includes phenolsulfonic acid, sulfosalicylic acid, and guaiacolsulfonic acid with no free acid is usable as a facial exfoliant or for use in a treatment for canker sores. A formulation that includes phenolsulfonic acid, sulfosalicylic acid, phenolsulfonate, and free acid is usable as a skin exfoliant for use on feet.

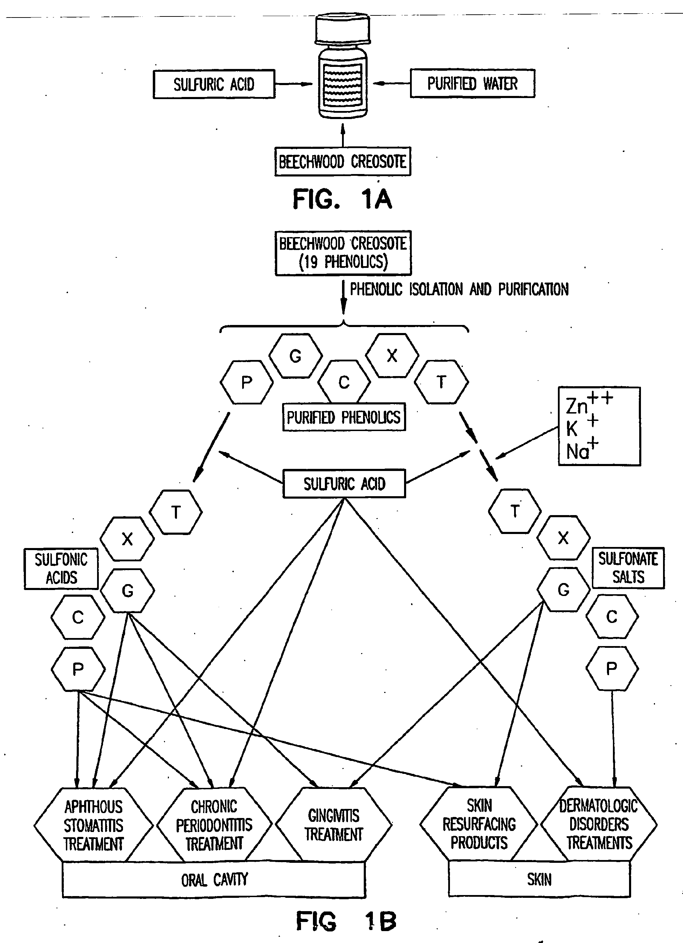

In its product aspect the present invention includes a formulation suitable for treating mucosal discontinuities that includes phenolsulfonic acid, sulfosalicylic acid, guaiacolsulfonic acid, and, for some embodiments, Ammonium phenolsulfonate, free sulfuric acid and water. In one embodiment, the formulation includes phenolic compounds that are isolated and purified, as is shown in FIG. 1B.

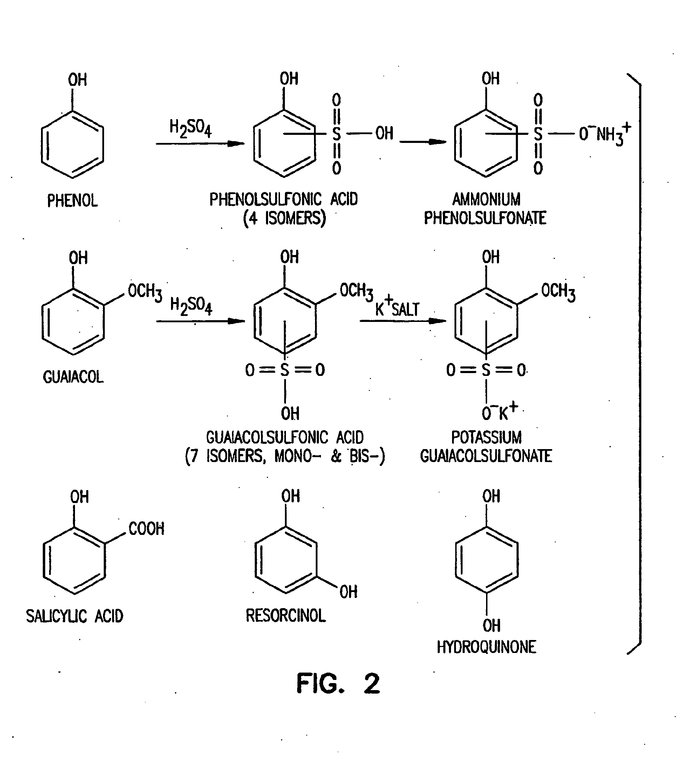

The purified phenolic compounds are shown in FIG. 2 and include phenol, guaiacol, salicylic acid, resorcinol and hydroquinone. The purified phenolic are treated with sulfuric acid to form sulfonic acids and sulfonate salts, as is shown in FIG. 1B. The sulfonate salts are treated with one or more of zinc, potassium and sodium to form a salt. Examples of other salts include alkali metal or alkaline earth metal salts and, particularly, calcium, magnesium, sodium, lithium, zinc, potassium, and iron salts. Phenolic compounds obtained from a variety of sources are usable to make the formulation of the present invention

Formulations that include the sulfonic acids of phenol and guaiacol may be used to treat mucosal discontinuities in the oral cavity such as aphthous stomatitis, canker sores, chronic periodontitis and gingivitis. Formulations that include the sulfonate salts of phenol and guaiacol may be used to treat mucosal discontinuities of the skin, such as dermatologic disorders. Formulations that include the phenolsulfonate salts also have use as skin resurfacing products. A breakdown of these acid and salt products is shown in FIG. 2.

When phenol is treated with sulfuric acid, the reaction products include four isomers of phenolsulfonic acid. When guaiacol is treated with sulfuric acid, the reaction products include seven isomers, a mono- and a bis- form of guaiacolsulfonic acid- Other sources of phenolsulfonic acid include the four isomers. Other sources of guaicol include the seven isomers, a mono- and a bis-form of guaiacolsulfonic acid.

The method and system of the present invention is a significant improvement over methods and systems used to make formulations prepared with Beechwood creosote because the method, system, and formulations of the present invention do not have the intense phenolic odor and taste of prior art products. Furthermore, the efficacy of the formulations of the present invention is very high because the acids and salts are separated from Beeehwood creosote and from each other or are synthesized in a process that does not require Beechwood creosote and are systematically and specifically blended in amounts that are targeted to treat the oral cavity and the skin, respectively.

One formulation embodiment may be used for treating mucosal discontinuities of the oral cavity. The formulation composition includes phenolsulfonic acid in a concentration of 30 percent by weight; guaiacolsulfonic acid in a concentration of 32 percent by weight; free sulfuric acid in a concentration of 24 percent by weight; water in a concentration of 11.9 percent by weight; and FD&C Red No. 40 in a concentration of 0.075 percent by weight. This formulation has a density of 1.59 g/mL, a total acidity of 9.1 mM/g, and a viscosity of 1025 cPs at 25 degrees Centigrade.

The formulation is applied with an applicator. The application contact time for treatment of periodontal disease is 5 to 30 seconds per infected tooth pocket. In one embodiment, the applicator is a pre-filled syringe. In another embodiment, the applicator is a kit that includes a topical anesthetic and an application device such as the pre-filled syringe. The topical local anesthetic is applied with any conventional applicator such as a cotton swab. As used herein, the term "local anesthetic" means any drug that provides local numbness or analgesia or any drug that provides a regional blockage of nociceptive pataways (afferent and/or efferent) and that is not an agonist or an antagonist of an opioid receptors. The local anesthetic can be any local anesthetic known or to be developed. Examples of local anesthetics suitable for use with the invention include: ambucaine, amolanone, amylcaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethisoquin, dimethocaine, diperodon, dyclonine, ecogonidine, ecogonine, euprocin, fenalcomine, formocaine, hexylcaine, hydroxyteteracaine, isobutyl p-aminobenzoate, leucinocaine, levoxadrol, lidocaine, mepivacaine, meprylcaine, metabutoxycaine, methyl chloride, myrtecaine, naepaine, octacaine, orthocaine, oxethazaine, parenthoxycaine, phenacaine, phenol, piperocaine, piridocaine, polidocanol, pramoxine, prilocaine, procaine, propanocaine, proparacaine, propipocaine, propoxycaine, pseudococaine, pyrrocaine, ropivacaine, salicyl alcohol, tetracaine, tolycaine, trimecaine, zolamine, or pharmaceutically-acceptable salts thereof, or mixtures thereof. The anesthetic is separately packaged for some embodiments and incorporated into the formulation for other embodiments.

Some kit and formulation embodiments include an analgesic. Examples of suitable analgesics include, but are not limited to, aceclofenac, acetaminophen, acetaminosalol, acetanilide, acetylsalicylsalicylic acid, alclofenac, alminoprofen, aloxiprin, aluminum bis(acetylsalicylate), aminochlorthenoxazin, 2-amino-4-picoline, aminopropylon, aminopyrine, ammonium salicylate, amtohnetin guacil, antipyrine, antipyrine salicylate, antrafenine, apazone, aspirin, benorylate, benoxaprofen, benzpiperylon, benzydamine, bermoprofen, bromfenac, p-bromoacetanilide, 5-bromosalicylic acid acetate, bucetin, bufexamac, bumadizon, butacetin, calcium acetylsalicylate, carbamazepine, carbiphene, carsalam, chlorthenoxazin(e), choline salicylate, cinchophen, ciramadol, clometacin, clonixin, cropropamide, crotethamide, dexoxadrol difenamizole, difiunisal, dihydroxyaluminum acetylsalicy, late, dipyrocetyl, dipyrone, emorfazone, enfenamic acid, epirizole, etersalate, ethenzamide, ethoxazene, etodolac, felbinac, fenoprofen, floctafenine, flufenamic acid, fluoresone, flupirtine, fluproquazone, flurbiprofen, fosfosal, gentisic acid, glafenine, ibufenac, imidazole salicylate, indomethacin, indoprofen, isofezolac, isoladol, isonixin, ketoprofen, ketorolac, p-lactophcnetide, lefetamine, lomoxicam, loxoprofen, lysine acerylsalicylate, magnesium acetylsalicylate, methotrimeprazine, metofoline, mofezolac, morazone, morpholine salicylate naproxen, nefopam, nifenazone, 5'-nitro-2'-propoxyacetanilide, parsalmide, perisoxal, phenacetin, phenazopyridine hydrochloride, phenocoll, phenopyrazone, phenyl acetylsalicylate, phenyl salicylate, phenyramidol, pipebuzone, piperylone, propacetamol, propyphenazone, ramifenazone, rimazolium metilsulfate, salacetamide, salicin, salicylamide, salicylamide o-acetic acid, salicylsulfuric acid, salsalate, salverine, simetride, sodium salicylate, suprofen, talniflumate, tenoxicam, terofenamate, tetrandrine, tinoridine, tolfenamic acid, tramadol, tropesin, viminol, xenbucin, and zomepirac

Acceptable component ranges for a formulation for treating mucosal discontinuities of the oral cavity, such as gingivitis, are as follows:

| Component | Concentration Ranges |
|---|---|
| Phenolsulfonic Acid | 25-80% by weight |
| Guaiacolsulfonic Acid | 25-80% by weight |
| Sulfosalicylic Acid | 0 -30% by weight |
| Citric Acid | 0-30% by weight |
| Ammonium Phenolsulfonate | 0-32% by weight |
| Free Sulfuric Acid | 0-32% by weight |
| Water | 13-30% by weight |

Acceptable component ranges for a formulation for treating mucosal discontinuities of the skin are as follows:

| **Component** | **Concentration Ranges** |
|---|---|
| Phenolsulfonic Acid | 25-80% by weight |
| Guaiacolsulfonic Acid | 25-80% by weight |
| Ammonium Phenolsulfonate | 0-5% by weight |
| Free Sulfuric Acid | 0-3% by weight |
| Water | 0-30% by weight |
| Colorant | 0.075-0.020% by weight |

These mucosal discontinuities include sores such as canker sores.

Another formulation embodiment of the present invention is suitable for use in the treatment of periodontal disease. Acceptable concentration ranges are as follows:

| **Component** | **Concentration Ranges** |
|---|---|
| Phenolsulfonic Acid | 25 to 80% |
| Guaiacolsulfonic Acid | 25 to 80% |
| Ammonium Phenolsulfonate | 0 to 32% |
| Free Sulfuric Acid | 0 to 32% |
| Water | 0 to 30% |
| Colorant | 0.00 to 0.075% |

The viscosity is 70 to 1000 cPs. The total acidity is 7.20 to 9.20 mM/g.
The density is 1.46 to 1.59 g/mL. The application time is 5 to at least 60 seconds. The applicator includes in some embodiments, a vial, a pre-filled syringe, or a swab or combinations of these applicators. The formulation optionally includes a colorant such as green or blue.

The formulation of the present invention has use as a liquid chemical cauterization agent for, in one embodiment, the oral cavity mucosa. The mechanisms of therapeutic action include solvent and keratolytic-based mechanisms that include penetrating and dissolving necrotic tissues. The mechanism also includes hygroscopic and dehydrating-based mechanisms. In particular, the formulation reduces tissue edema. The solvation is exothermic. The solvation releases acidification. The mechanism is also a denaturant and keratocoagulant-based. The formulation causes acidification necrosis and oxidation. The formulation also aids in generation of a surrogate Eschar /clot that acts as a protective natural bandage.

The formulation of the present invention provides instantaneous pain relief to a user and accelerates ulcer healing. The formulation kills infectious organisms and is effective in a single application applied to an ulcer or other mucosal discontinuity. The formulation of the present invention is self-limited and is not harmful to healthy mucosa. The reaction of the formulation is limited by water solvation, barrier membrane formation, and acid neutralization.

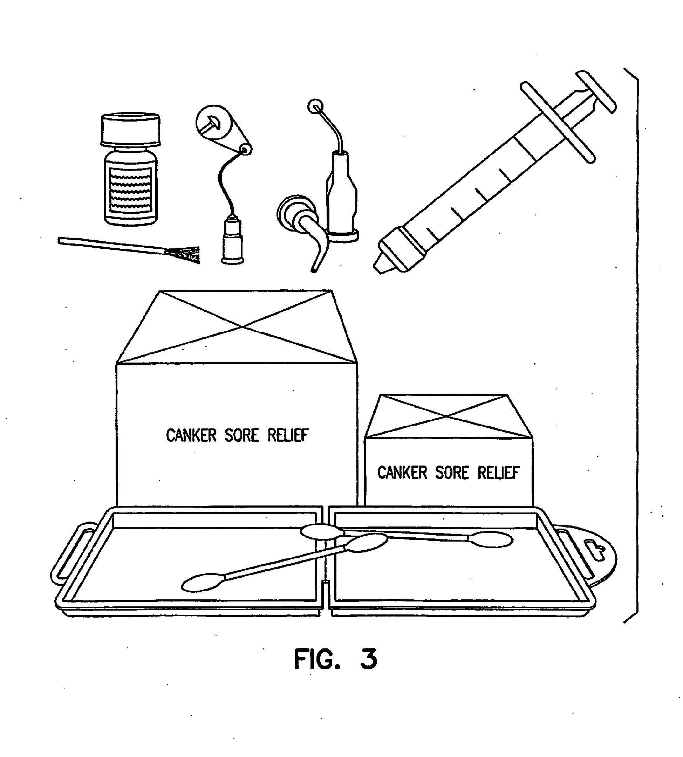

Devices for applying the formulations of the present invention are shown in FIG. 3 and include a syringe and a cotton swab. In one embodiment, the syringe is prefilled. In one embodiment, the formulation of the present invention is enclosed within a swab device. In another embodiment, the formulation is enclosed in a container with a delivery mechanism that delivers the formulation drop-by-drop. In another embodiment, the formulation is metered. In one other embodiment, the formulation is enclosed in a container that includes a mechanism for heating or cooling the formulation. The mechanism includes chemicals, separated in compartments by a breakable seal, adjacent to the formulation of the present invention, that create either an exothermic or endothermic reaction when combined. In one embodiment, the chemicals are separated from the formulation by a wall. The chemicals are combined when a user breaks the breakable seal. The heating or cooling is transferred to the formulation through the wall. The packaged formulation of the present invention is transported for sale to pharmacies and drug stores. In other embodiments, the formulation of the present invention is packaged with an anesthetic. The anesthetic is applied to the mucosal discontinuity first.

In its method aspect, one embodiment of the present invention includes a method for preparing treatments suitable for mucosal discontinuities in the oral cavity and skin. One method embodiment is described in the following example:

### EXAMPLE 1

Phenolsulfonic acid solution was prepared in sulfuric acid. The method included setting up a recirculating water bath so that the water was at a temperature of 95 degrees Centigrade. Next, the weight of available stock of Liquefied Phenol USP containing 3.23 moles of Phenol was calculated. Phenol has a molecular weight of 94.11. The weight of 3.23 moles of Phenol is 3.23 x 94.11 + 303.97 grams. The purity of Liquefied Phenol USP typically ranges from 89.0% to 91.5%. Using the purity value either from the manufacturer's Certificate of Analysis or the purity value from an internal quality control analysis, the weight of the available stock of Liquefied Phenol USP required to deliver 3.23 moles of Phenol was calculated.

The amount of available stock sulfuric acid (NF) that contains 7.08 moles was calculated. The purity of sulfuric acid NF typically ranges from 95.0% to 98%. A 1 liter Corning Pyrex Bottle No. 1395 was placed on a Mettler PG-5002-S balance. A 600 mL beaker was filled with about 400 mL of Liquefied Phenol USP from the stock bottle. A quantity of 3.23 moles of phenol as liquefied phenol USP by weight was added from the beaker using the weight of available stock. The amount of liquid phenol was calculated to provide 3.23 moles of phenol.

About 400 mL of stock sulfuric acid NF were added to a 600 mL Pyrex beaker. With the 1 liter Corning bottle containing the Liquefied Phenol still on the Mettler balance, 7.08 moles of sulfuric acid was added to the bottle as stock sulfuric acid NF by weight from the beaker using the weight of available stock sulfuric acid NF that was calculated to provide 7.08 moles of sulfuric acid.

A 5 cm magnetic stirring bar was placed in the bottle containing the phenol/sulfuric acid mixture. The bottle and mixture were enclosed with a standard Corning low-temperature closure, placed on the bottle. The mixture was mixed on a magnetic stir plate for thirty minutes. The bottle was placed in a pre-heated 95 degrees Centigrade recirculating water bath for 14 to 24 hours.

The bottle was removed from the water bath after 14 to 24 hours. The bottle was allowed to cool to room temperature and an identifying sticker was applied.

A solution of guaiacolsulfonic acid in sulfuric acid was prepared. Water in the recirculating water bath was heated to 65 degrees Centigrade. The weight of available stock guaiacol, purified reagent, that contained 1.63 moles of guaiacol was calculated. The amount of available stock of sulfuric acid NF that contained 3.41 moles of sulfuric acid was calculated. A1 liter Corning Pyrex bottle No. 1395 was placed on the Mettler PG-5002-S balance. A 600 mL beaker was filled with approximately 400 mL guaiacol, purified reagent from the available stock bottle. A quantity of 1.63 moles of guaiacol as guaiacol, purified reagent by weight from the beaker was added to the bottle.

A 5 cm magnetic stir bar was placed in the bottom of the Pyrex bottle. The bottle was placed on a magnetic stir plate and stirred at low speed. A ring stand was placed next to the stir plate. A thermometer was mounted onto the ring stand so that the tip of the thermometer was positioned in the guaiacol. A 250 mL cylinder was also mounted onto the ring stand so that the tip was inserted into the opening of the Pyrex bottle.

A 600 mL Pyrex beaker was filled with approximately 400 mL of stock sulfuric acid NF. A second 600 mL beaker was placed on the Mettler PG-5002-S balance. A quantity of 3.41 moles of sulfuric acid as stock sulfuric acid NF was added to the second beaker. With the cylinder stopcock in the closed position, the weighed amount of sulfuric acid NF was transferred from the second beaker to the addition cylinder mounted above the Pyrex bottle containing the measured quantity of guaiacol. The sulfuric acid was added from the addition cylinder to the guaiacol in the bottle in 10 to 20 mL aliquots while maintaining steady stirring. The temperature of the mixture was monitored to maintain a range of 55 to 65 degrees Centigrade until all of the acid was placed in the bottle.

The reaction mixture was stirred for 30 minutes after all of the sulfuric acid was added to the mixture. The mixture was enclosed by a standard Corning low temperature closure on the bottle. The bottle was placed in a pre-heated 65 degrees Centigrade recirculating water bath for 14 to 24 hours. The bottle was removed after the 14 to 24 hours and allowed to come to room temperature. The bottle was then marked.

The phenolsulfonic acid solution and the guaiacolsulfonic acid solution were then subjected to quality control. After being release by Quality Control, 1000 grams of the phenolsulfonic acid solution and 500 grams of the guaiacolsulfonic acid solution were prepared in a 2 liter Corning Pyrex bottle No. 1395 using the Mettler PG-5002-S balance. A quantity of 1.125 grams of FD&C Red #40 powdered colorant was weighed and added to the phenolsulfonic/guaiacolsulfonic mixture. The bottle was mixed by inverting repeatedly until the color was evenly dispersed. The colored phenolsulfonic/guaiacolsulfonic mixture was then available for use.

### EXAMPLE 2

A facial exfoliant was prepared. A solution of phenolsulfonic acid was prepared as described in Example 1, except that the weight of available stock liquefied phenol USP was calculated and prepared for 4.0 moles of phenol. The amount of available stock sulfuric acid NF that contained 4.0 moles was calculated. The weight of sulfuric acid NF was 392.32 grams. The 4.0 moles of sulfuric acid NF was treated as described in Example 1. A quantity of 400 mL of Liquefied Phenol USP was added to a 600 mL beaker. A quantity of 4.0 moles of phenol as Liquefied Phenol USP was added to a 1L Corning Pyrex No. 1395 bottle using the Mettler PS-5002-S balance.

A second 600 mL Pyrex beaker was filled with approximately 400 mL of stock sulfuric acid NF. With the 1 L Corning bottle containing the liquefied phenol still on the Mettler balance, 4.0 moles of sulfuric acid as stock sulfuric acid NF by weight, was added to the bottle.

A 5 cm magnetic stirring bar was placed in a bottle containing the phenol/sulfuric acid mixture and enclosed with a Corning low-temperature closure. The solution was mixed for 30 minutes. The bottle was then placed in a pre-heated 95 degree Centigrade recirculating water bath for 14 to 24 hours. After 14 to 24 hours, the bottle was removed and allowed to come to room temperature and marked with a sticker.

The weight of available stock guaiacol, purified reagent that contained 3.50 moles of guaiacol was calculated. The amount of available stock sulfuric acid NF that contained 3.50 moles of sulfuric acid was also calculated. A 1L Corning Pyrex bottle No. 1395 was placed on the Mettler PG-5002-S balance. A 600 mL beaker was filled with approximately 400 mL of guaiacol, purified reagent. A quantity of 3.50 moles of guaiacol was added from the beaker to the 1L Pyrex bottle. A quantity of 3.50 moles of sulfuric acid NF was added to the 1L Pyrex bottle, as described in Example 1, in 10 to 20 mL aliquots while maintaining steady stirring between 55 to 65 degrees Centigrade until all of the acid had been placed in the bottle. The mixture was continuously stirred for 30 minutes after all of the sulfuric acid has been added and a Corning low temperature closure was placed on the bottle. The bottle was placed in a preheated 65 degree Centigrade recirculating water bath for 14 to 24 hours. The bottle was removed from the water bath after 14 to 24 hours. The bottle was allowed to come to room temperature and was then marked for quality control.

A quantity of 750 grams of the phenolsulfonic acid solution and 750 grams of the guaiacolsulfonic acid solution were combined in a 2L Corning Pyrex bottle. A quantity of 1.5 grams of Citronellal fragrance was added. The acids were mixed by inverting the bottle repeatedly until evenly blended.

The method also includes treating the purified phenolics with sulfuric acid to make sulfonic acids and sulfonate salts. The method further includes the suitability of the sulfonic acids to treat mucosal discontinuities in the oral cavity such as aphthous stomatitis, chronic periodontitis, canker sores, and gingivitis. The method includes the suitability of the sulfonate salts to treat dermatologic disorders and for skin resurfacing.

### EXAMPLE 3 (not according to the invention)

A skin exfoliant for use on feet was prepared. A solution of phenolsulfonic acid was prepared as described in Example 1, except that the weight of available stock liquefied phenol USP was calculated and prepared for 5.0 moles of phenol. The amount of available stock sulfuric acid NF that contained 5.0 moles was calculated. The weight of sulfuric acid NF was 490.4 grams. The 5.0 moles of sulfuric acid NF was treated as described in Example 1. A quantity of 470.55 gms of Liquefied Phenol USP was added to a 600 mL beaker. A quantity of 5.0 moles of phenol as Liquefied Phenol USP was added to a 1L Corning Pyrex No. 1395 bottle using the Mettler PS-5002-S balance.

A second 600 mL Pyrex beaker was filled with approximately 400 mL of stock sulfuric acid NF. With the 1 L Corning bottle containing the liquefied phenol still on the Mettler balance, 5.0 moles of sulfuric acid as stock sulfuric acid NF by weight, was added to the bottle.

A 5 cm magnetic stirring bar was placed in a bottle containing the phenol/sulfuric acid mixture and was enclosed with a Corning low-temperature closure. The solution was mixed for 30 minutes. The bottle was then placed in a pre-heated 95 degree Centigrade recirculating water bath for 14 to 24 hours. After 14 to 24 hours, the bottle was removed and was allowed to come to room temperature and marked with a sticker.

The phenolsulfonic acid and beaker were placed on the Mettler balance. A quantity of 300 grams of reagent grade ammonium phenolsulfonate was added to the phenolsulfonic acid. A quantity of 30 grams of reagent grade zinc phenolsulfonate was added to the phenolsulfonic acid. A quantity of 1.5 grams of Citronellal fragrance was added. The acids were mixed by vigorous stirring using a power hand blender.

### EXAMPLE 4

A treatment for canker sores was formulated. A solution of phenolsulfonic acid was prepared as described in Example 1, except that the weight of available stock liquefied phenol USP was calculated and prepared for 2.4 moles of phenol. The amount of available stock sulfuric acid NF that contained 5.26 moles was calculated. The weight of sulfuric acid NF was 515.90 grams. The 5.26 moles of sulfuric acid NF was treated as described in Example 1. A quantity of 400 mL of Liquefied Phenol USP was added to a 600 mL beaker. A quantity of 2.4 moles of phenol as Liquefied Phenol USP was added to a 1L Corning Pyrex No. 1395 bottle using the Mettler PS-5002-S balance.

A second 600 mL Pyrex beaker was filled with approximately 400 mL of stock sulfuric acid NF. With the 1 L Corning bottle containing the liquefied phenol still on the Mettler balance, 5.26 moles of sulfuric acid as stock sulfuric acid NF by weight, was added to the bottle.

A 5 cm magnetic stirring bar was placed in a bottle containing the phenol/sulfuric acid mixture and enclosed with a Corning low-temperature closure. The solution was mixed for 30 minutes. The bottle was then placed in a pre-heated 95 degree Centigrade recirculating water bath for 14 to 24 hours. After 14 to 24 hours, the bottle was removed and allowed to come to room temperature and marked with a sticker.

The weight of available stock guaiacol, purified reagent that contains 2.3 moles of guaiacol was calculated. The amount of available stock sulfuric acid NF that contains 4.82 moles of sulfuric acid was also calculated. A 1L Corning Pyrex bottle No. 1395 was placed on the Mettler PG-5002-S balance. A 600 mL beaker was filled with approximately 400 mL of guaiacol, purified reagent. A quantity of 2.3 moles of guaiacol was added from the beaker to the 1L Pyrex bottle. A quantity of 4.82 moles of sulfuric acid NF was added to the 1L Pyrex bottle, as described in Example 1, in 10 to 20 mL aliquots while maintaining steady stirring between 55 to 65 degrees Centigrade until all of the acid had been placed in the bottle. Stirring was continued for 30 minutes after all of the sulfuric acid has been added and a Corning low temperature closure was placed on the bottle. The bottle was placed in a pre-heated 65 degree Centigrade recirculating water bath for 14 to 24 hours. The bottle was removed from the water bath after 14 to 24 hours. The bottle was allowed to come to room temperature and then was marked for quality control.

A quantity of 750 grams of the phenolsulfonic acid solution and 750 grams of the guaiacolsulfonic acid solution were combined in a 2L Corning Pyrex bottle. A quantity of 1.5 grams of Citronellal fragrance was added. The acids were mixed by inverting the bottle repeatedly until evenly blended.

The method also includes treating the purified phenolics with sulfuric acid to make sulfonic acids and sulfonate salts. The method further includes the suitability of the sulfonic acids to treat mucosal discontinuities in the oral cavity such as aphthous stomatitis, chronic periodontitis and gingivitis. The method includes the suitability of the sulfonate salts to treat dermatologic disorders and for skin resurfacing.

Another embodiment of the present invention includes an exfoliation composition. The exfoliation composition of the present invention includes creams, gels, foams and pastes. The formulation includes a diluent such as water, aqueous alcohol, glycol or other inactive carrier which includes up to about 4 percent sulfonated phenol exfoliating material. The exfoliation material, intended for topical application includes, for some embodiments, carrier, excipient, or vehicle ingredients such as, for example, water, acetone, ethanol, ethylene glycol, propylene glycol, butane-1,3 diol, isopropyl myristate, isopropyl palmitate, mineral oil and mixtures thereof to form lotions, tinctures, creams, emulsions, gels, or ointments which are non-toxic and pharmaceutically, cosmetically or dermatologically acceptable. Additionally, moisturizers or humectants are added to the present composition, if desired.

In addition to the diluent, formulations of the sulfonated phenols also, for some embodiments, include other standard adjuvants such as an emollient, moisturizer, thickener, emulsifier, neutralizer, coloring agent, UV absorber or filter, preservative and or gelling agent. When employed in a formulation, these adjutants are present in amounts ranging from about 0.5% to 30%.

### Emollient

Acceptable emollients include saturated fatty acids such as isopropyl myristate, cetyl palmitate and octyldodecylmyristate, beeswax, saturated and unsaturated fatty alcohols such as behenyl alcohol and cetyl alcohol, hydrocarbons such as mineral oils, petrolatum, squalene, fatty sorbitan esters, lanolin and lanolin derivatives, such as lanolin alcohol ethoxylated, hydroxylated and acetylated lanolins, cholesterol and derivatives thereof, animal and vegetable triglycerides such as almond oil, peanut oil, wheat germ oil, linseed oil, jojoba oil, oil of apricot pits, walnuts, palm nuts, pistachio nuts, sesame seeds, rapeseed, cade oil, corn oil, peach pit oil, poppyseed oil, pine oil, castor oil, soybean oil, avocado oil, safflower oil, coconut oil, hazelnut oil, olive oil, grapeseed oil, and sunflower seed oil and diisostearylmalate, diisostearyldimerate and triisostearyltrimerate.

Suitable emollients for use herein include isocetyl alcohol, octyl palmitate, isostearyl neopentanoate and isocetyl stearyl stearate, natural or synthetic oils selected from mineral, vegetable, and animal oils, fats and waxes, fatty acid esters, fatty alcohols, alkylene glycol and polyalkylene glycol ethers and esters, fatty acids and mixtures thereof.

### Emulsifier

Suitable emulsifiers include glyceryl stearate and laureth 23, PEG 20 stearate, and mink-amidopropyl dimethyl 2-hydroxyethylammonium chloride.

Typical moisturizers used in the formulation of the present invention include glycerin, petrolatum and maleated vegetable oil.

The sulphonated phenolic material is formulated, in some embodiments, with a gelling agent. Suitable gelling agents include water soluble or colloidally water soluble polymers and include cellulose ethers, such as hydroxyethyl cellulose, methyl cellulose, hydroxypropylmethyl cellulose, polyvinylalcohol, polyquaternium-10, guar gum, hydroxypropyl guar gum and xanthan gum. Other gelling agents usable the present invention include acrylic acid/ethyl acrylate copolymers and carboxyvinyl polymers. Also usable are maleic anhydride-alkyl methylvinylethers and copolymers, natural gums, and polymethacrylate copolymer. Other suitable gelling agents include oleogels such as trihydroxystearin and aluminum magnesium hydroxy stearate.

Some embodiments of the formulation of the present invention include preservatives. The preservatives include sodium benzoate and propyl paraben and mixtures of these materials. Other additional materials include fragrances, fillers such as nylon, sun screens, electrolytes such as sodium chloride, proteins, antioxidants and chelating agents, and ultraviolet absorbing agents. The ultraviolet absorbing agents include benzophenone-3, benzophenone-4, oxytyl dimethyl PABA (Padimate O), octyl methoxy cinnamate, octyl salicylate, octocrylene, p-methylbenzylidene camphor, butyl methoxy dibenzoyl methane, titanium dioxide, zinc oxide and mixtures of these materials.

The exfoliant compositions of the present invention are believed to enhance cell renewal, skin smoothing, exfoliation of calloused skins, removal of skin blemishes, reduction in corn size, and in toning skin.

In addition to these and other vehicles which are selected by those being skilled in the art, it is understood that pharmaceutical and cosmetic compositions of the present invention include other ingredients such as, for example, those that improve or eradicate age spots, keratosis and wrinkles; analgesics; anaesthetics; anti-acne agents; anti-bacterial, anti-yeast agents, anti-fungal agents, anti-viral agents, anti-dandruff agents, anti-dermatitis agents, anti-pruritic agents, anti-metic agents, anti-inflammatory agents, anti-hyperkeratolytic agents, anti-dry skin agents, anti-perspirants, anti-psoriatic agents, anti-seborrheic agent, hair conditioners and hair treatment agents, anti-aging and anti-wrinkle agents, skin-whitening agents, de-pigmenting agents, vitamins, tanning agents, hormones, retinoids, vitamin A palpitate and vitamin E acetate.

The present invention may also be used in methods for enhancing epidermal exfoliation and/or enhancing epidermal skin renewal. The method includes topically administering to an area of a subject's skin, an effective amount of a composition of the present invention for a period of time effective to enhance epidermal exfoliation and /or enhance epidermal skin renewal.

The present invention may also be used in methods for improving the texture and/or appearance of skin. The method comprises administering to an area of a subject's skin an effective amount of the exfoliant of the present invention for a period of time effective to improve the texture and/or appearance of skin. In one embodiment, the application time is within a range of thirty seconds to shirty minutes.

The present invention may also be used in methods for treating or preventing an abnormal skin condition, disease or disorder. The methods comprise administering to an area of a subject's skin an effective amount of the exfoliant of the present invention which comprises two isomers of phenolsulfonic acid, four isomers of gualacolsulfonic acid and, optionally, sulfosalicylic acid for a period of time effective to treat or prevent an abnormal skin condition, disease, or disorder. The time range is thirty seconds to thirty minutes.

The condition, disease or disorder includes, but is not limited to, dry skin, severe dry skin, dandruff, acne, keratosis, eczema, skin flakiness, age spots, hyper-pigmented skin, inflammatory dermatosis, age-related skin changes, skin in need of cleansers, and the effects of skin atrophy and psoriasis.

One method of administration of an effective amount of the exfoliant of the present invention for any of the methods described herein is when on an area of skin by a topical application. The amount of the exfoliant and frequency of topical application to the skin varies widely, depending upon factors such as the particular skin disorder, the severity of the skin disorder, the location and/or type of skin involved, the subject's skin sensitivity, and the degree of treatment desired. It is well within the purview of the skilled artisan to administer regular dosages according to a subject's need. It is suggested as an example that daily application range from about once per week to about one time per day.

Another mode of administration is a chronic administration. Chronic administration has a duration of months to years. Chronic administration also ranges from about once per week to once per day.

A kit embodiment suitable for use in the treatment of calluses on feet or other body part, includes the exfoliation formulation of the present invention, a container for the formulation and a device for abrading a callous after the exfoliation formulation is applied. The device includes one or more of an abrasive pad, a brush, an abrasive board, or other abrasive device.

Some embodiments of the exfoliant are applied in spas, salons, or other facilities specializing in skin care. Other embodiments are applied in the home by a purchaser. Some embodiments of the exfoliant are sold in drug stores, grocery stores, and department stores.

## Claims

1. A method for making a formulation suitable for treating mucosal discontinuities, comprising:
providing phenol, guaiacol, sulfuric acid and, optionally, water in preselected concentrations;
reacting the guaiacol and phenol with the sulfuric acid and, optionally, water to make phenolsulfonic acid and guaiacolsulfonic acid and, optionally, combining with free water and free acid to produce a formulation having a preselected concentration of preselected isomers of phenolsulfonic acid, preselected isomers of gualacolsulfonic acid, and optionally, free acid and free water.

2. The method of claim 1, wherein the phenolsulfonic acid is further treated with ammonium hydroxide to make ammonium phenolsulfonate.

3. The method of claim 1, wherein the guaiacolsulfonic acid is further treated with potassium hydroxide to make potassium guaiacolsulfonate or zinc hydroxide to make zinc guaiacolsulfonate.

4. The method of claim 2 or 3, further comprising separating sulfonic acids from sulfonate salts.

5. The method of claim 4, further comprising packaging the phenolsulfonic acid suitable to treat one or more of aphthous stomatitis, chronic periodontitis, and gingivitis.

6. The method of claim 4, further comprising packaging sulfonate salts suitable to treat one or more of dermatologic disorders and for skin resurfacing or canker sores.

7. A product produced by the method of claim 1.

8. A product produced by the method of claim 3.

9. A formulation suitable for use in the treatment of mucosal discontinuities, comprising: isomers of phenolsulfonic acid, isomers of guaiacolsulfonic acid, and optionally, free acid and free water, the formulation being obtainable by the method of claim 1.

10. A formulation suitable for use in the treatment of mucosal discontinuities, comprising: phenolsulfonic acid and isomers of phenolsulfonic acid; guaiacolsulfonic acid and isomers of guaiacolsulfonic acid and mono- and bis-forms of guaiacolsulfonic acid; ammonium phenolsulfonate; and potassium guaiacolsulfonate, the isomers of phenolsulfonic acid and of guaiacolsulfonic acid being obtainable by the method of claim 1.

11. A formulation suitable for use in the treatment of oral mucosal discontinuities, comprising: phenolsulfonic acid in a concentration of 25-80% by weight; guaiacolsulfonic acid in a concentration of 25-80% by weight; free sulfuric acid in a concentration of 0 to 32% by weight ; and water in a concentration of 0 to 3% by weight.

12. A formulation suitable for use in the treatment of skin mucosal discontinuities, comprising: phenolsulfonic acid in a concentration of 25-80% by weight; ammonium phenolsulfonate in a concentration of 0 to 5% by weight; guaiacolsulfonic acid in a concentration of 25-80% by weight; free sulfuric acid in a concentration of 0 to 32% by weight; and water in a concentration of 0 to 3% by weight.

13. A kit suitable for use in the treatment of mucosal discontinuities comprising the formulation of claim 10, 11 or 12, a container for containing the formulation and a mechanism for delivering the formulation to a mucosal discontinuity.

14. A device suitable for use in the treatment of mucosal discontinuities, comprising:
A syringe, and
The product of claim 7 contained in the syringe.

15. A system comprising:
Ingredients that include phenolsulfonic acid, guaiacolsulfonic acid, ammonium phenolsulfonate, potassium guaiacolsulfonate, water and free acid; and
a mechanism calibrated to mix preselected amounts of two or more of the ingredients together to make a formulation suitable for use in the treatment of a specific type of mucosal discontinuity.

16. The system of claim 15, further comprising one or more of salicylic acid and sulfosalicylic acid.

17. The system of claim 15, wherein the phenolsulfonic acid, guaiacolsulfonic acid, ammonium phenolsulfonate, potassium guaiacolsulfonate are metered to prepare a formulation suitable for treating gingivitis, wherein the formulation includes little or no free acid.

18. The system of claim 15, wherein the phenolsulfonic acids and guaiacolsulfonic acid are metered to form a formulation with free sulfuric acid, wherein the formulation is useful to treat periodontal disease.

19. The system of claim 15, wherein the phenolsulfonic acid and guaiacolsulfonic acid are metered, in concentrations effective for use as a facial exfoliant or as a treatment for canker sores.

20. The system of claim 15, wherein the phenolsulfonic acid, phenolsulfonate, and free acid are metered in concentrations effective for use as an exfoliant for feet.

21. An exfoliating composition, comprising: a mixture comprising phenolsulfonic acid, guaiacolsulfonic acid and, optionally, sulfosalicylic acid.

22. The exfoliating composition of claim 21, further comprising one or more of an emollient, a moisturizer, a thickener, and emulsifier, a neutralizer, a coloring agent, and UV absorber or filter, and a preservative.

23. The exfoliating composition of claim 21, wherein the mixture is a gel.

24. The exfoliating composition of claim 21, wherein the mixture is a cream or paste or foam.

25. The exfoliating composition of claim 21, wherein the mixture has a pH within a range of 2 to 6.

26. The exfoliating composition of claim 21, wherein the phenol sulfonic acid has a concentration up to at least 24% by volume of the mixture.

27. The exfoliating composition of claim 21, wherein the phenol sulfonic acid concentration is 4% by volume of the mixture.

28. The exfoliating composition of claim 21, further comprising citric acid.

## Patentansprüche

1. Verfahren zur Herstellung einer Formulierung, die für die Behandlung von Schleimhautdiskontinuitäten geeignet ist, umfassend:
das Bereitstellen von Phenol, Guaiacol, Schwefelsäure und wahlweise Wasser in vorgewählten Konzentrationen,
das Reagieren des Guaiacols und Phenols mit der Schwefelsäure und wahlweise Wasser, um Phenolsulfonsäure und Guaiacolsulfonsäure herzustellen, und wahlweise das Kombinieren mit freiem Wasser und freier Säure, um eine Formulierung herzustellen, die eine vorgewählte Konzentration vorgewählter Isomere von Phenolsulfonsäure, vorgewählter Isomere von Guaiacolsulfonsäure und wahlweise freie Säure und freies Wasser aufweist.

2. Verfahren nach Anspruch 1, wobei die Phenolsulfonsäure des Weiteren mit Ammoniumhydroxid behandelt wird, um Ammoniumphenolsulfonat herzustellen.

3. Verfahren nach Anspruch 1, wobei die Guaiacolsulfonsäure des Weiteren mit Kaliumhydroxid, um Kaliumguaiacolsulfonat herzustellen, oder Zinkhydroxid, um Zinkguaiacolsulfonat herzustellen, behandelt wird.

4. Verfahren nach Anspruch 2 oder 3, des Weiteren das Trennen von Sulfonsäuren von Sulfonatsalzen umfassend.

5. Verfahren nach Anspruch 4, des Weiteren das Verpacken der Phenolsulfonsäure umfassend, die zum Behandeln einer oder mehrerer von Mundfäule, chronischer Parodontitis und Zahnfleischentzündung geeignet ist.

6. Verfahren nach Anspruch 4, des Weiteren das Verpacken von Sulfonatsalzen umfassend, die zum Behandeln von einer oder mehreren dermatologischen Beschwerden und zur Hautverjüngung oder gegen Aphthe geeignet sind.

7. Produkt, das durch das Verfahren nach Anspruch 1 hergestellt wird.

8. Produkt, das durch das Verfahren nach Anspruch 3 hergestellt wird.

9. Formulierung, die zur Verwendung bei der Behandlung von Schleimhautdiskontinuitäten geeignet ist, umfassend: Isomere von Phenolsulfonsäure, Isomere von Guaiacolsulfonsäure und wahlweise frei Säure und freies Wasser, wobei die Formulierung durch das Verfahren nach Anspruch 1 erhältlich ist.

10. Formulierung, die zur Verwendung bei der Behandlung von Schleimhautdiskontinuitäten geeignet ist, umfassend: Phenolsulfonsäure und Isomere von Phenolsulfonsäure; Guaiacolsulfonsäure und Isomere von Guaiacolsulfonsäure und
Mono- und Bis-Formen von Guaiacolsulfonsäure; Ammoniumphenolsulfonat; und Kaliumguaiacolsulfonat, wobei die Isomere von Phenolsulfonsäure und von Guaiacolsulfonsäure durch das Verfahren nach Anspruch 1 erhältlich sind.

11. Formulierung, die zur Verwendung bei der Behandlung von oralen Schleimhautdiskontinuitäten geeignet ist, umfassend: Phenolsulfonsäure in einer Konzentration von 25-80 Gew.-%; Guaiacolsulfonsäure in einer Konzentration von 25-80 Gew.-%; freie Schwefelsäure in einer Konzentration von 0 bis 32 Gew.-%; und Wasser in einer Konzentration von 0 bis 3 Gew.-%.

12. Formulierung, die zur Verwendung bei der Behandlung von Hautschleimhautdiskontinuitäten geeignet ist, umfassend: Phenolsulfonsäure in einer Konzentration von 25-80 Gew.-%; Ammoniumphenolsulfonat in einer Konzentration von 0 bis 5 Gew.-%; Guaiacolsulfonsäure in einer Konzentration von 25-80 Gew.-%; freie Schwefelsäure in einer Konzentration von 0 bis 32 Gew.-%; und Wasser in einer Konzentration von 0 bis 3 Gew.-%.

13. Kit, das zur Verwendung bei der Behandlung von Schleimhautdiskontinuitäten geeignet ist, umfassend die Formulierung nach Anspruch 10, 11 oder 12, einen Behälter zum Halten der Formulierung und einen Mechanismus zum Abgeben der Formulierung an eine Schleimhautdiskontinuität.

14. Vorrichtung, die zur Verwendung bei der Behandlung von Schleimhautdiskontinuitäten geeignet ist, umfassend:
eine Spritze und
das Produkt nach Anspruch 7, das in der Spritze gehalten wird.

15. System umfassend:
Bestandteile, die Phenolsulfonsäure, Guaiacolsulfonsäure, Ammoniumphenolsulfonat, Kaliumguaiacolsulfonat, Wasser und freie Säure umfassen;
und
einen Mechanismus, der zum Zusammenmischen vorgewählter Mengen von zwei oder mehreren der Bestandteile kalibriert ist, um eine Formulierung herzustellen, die für die Verwendung bei der Behandlung eines spezifischen Typs von Schleimhautdiskontinuität geeignet ist.

16. System nach Anspruch 15, des Weiteren eine oder mehrere von Salicylsäure und Sulfosalicylsäure umfassend.

17. System nach Anspruch 15, wobei die Phenolsulfonsäure, Guaiacolsulfonsäure, das Ammoniumphenolsulfonat, Kaliumguaiacolsulfonat dosiert werden, um eine Formulierung herzustellen, die zum Behandeln von Zahnfleischentzündung geeignet ist, wobei die Formulierung kaum eine oder keine freie Säure umfasst.

18. System nach Anspruch 15, wobei die Phenolsulfonsäuren und Guaiacolsulfonsäure dosiert werden, um eine Formulierung mit freier Schwefelsäure zu bilden, wobei die Formulierung zum Behandeln einer parodontalen Erkrankung geeignet ist.

19. System nach Anspruch 15, wobei die Phenolsulfonsäure und Guaiacolsulfonsäure in Konzentrationen dosiert werden, die zur Verwendung als Gesichtspeeling oder als Behandlung von Aphthe wirksam sind.

20. System nach Anspruch 15, wobei die Phenolsulfonsäure, das Phenolsulfonat und die freie Säure in Konzentrationen dosiert werden, die zur Verwendung als Peeling für die Füße wirksam sind.

21. Peelingzusammensetzung umfassend: eine Mischung umfassend Phenolsulfonsäure, Guaiacolsulfonsäure und wahlweise Sulfosalicylsäure.

22. Peelingzusammensetzung nach Anspruch 21, des Weiteren eines oder mehrere von einem Emolliens, einer Feuchtigkeitscreme, einem Verdickungsmittel und Emulgator, einem Neutralisationsmittel, einem Farbmittel und UV-Absorber oder Filter und einem Konservierungsmittel umfassend.

23. Peelingzusammensetzung nach Anspruch 21, wobei die Mischung ein Gel ist.

24. Peelingzusammensetzung nach Anspruch 21, wobei die Mischung eine Creme oder Paste oder ein Schaummittel ist.

25. Peelingzusammensetzung nach Anspruch 21, wobei die Mischung einen pH-Wert im Bereich von 2 bis 6 aufweist.

26. Peelingzusammensetzung nach Anspruch 21, wobei die Phenolsulfonsäure eine Konzentration von bis zu mindestens 24 Volumen-%, auf die Mischung bezogen, aufweist.

27. Peelingzusammensetzung nach Anspruch 21, wobei die Phenolsulfonsäurekonzentration 4 Volumen-%, auf die Mischung bezogen, beträgt.

28. Peelingzusammensetzung nach Anspruch 21, des Weiteren Zitronensäure umfassend.

## Revendications

1. Procédé de préparation d'une formulation adaptée au traitement des discontinuités des muqueuses, comprenant les étapes consistant à:
se procurer du phénol, du gaïacol, de l'acide sulfurique et, éventuellement, de l'eau, à des concentrations prédéfinies;
faire réagir le gaïacol et le phénol avec l'acide sulfurique et, éventuellement, l'eau pour préparer de l'acide phénolsulfonique et de l'acide gaïacolsulfonique et, éventuellement, combiner avec l'eau libre et l'acide libre pour produire une formulation ayant une concentration prédéfinie d'isomères prédéfinis de l'acide phénolsulfonique, d'isomères prédéfinis de l'acide gaïacolsulfonique, et éventuellement, d'acide libre et d'eau libre.

2. Procédé selon la revendication 1, dans lequel l'acide phénolsulfonique subit un traitement supplémentaire avec de l'hydroxyde d'ammonium pour préparer du phénolsulfonate d'ammonium.

3. Procédé selon la revendication 1, dans lequel l'acide gaïacolsulfonique subit un traitement supplémentaire avec de l'hydroxyde de potassium pour préparer du gaïacolsulfonate de potassium ou avec de l'hydroxyde de zinc pour préparer du gaïacolsulfonate de zinc.

4. Procédé selon la revendication 2 ou 3, comprenant en outre l'étape consistant à séparer les acides sulfoniques des sels sulfonates.

5. Procédé selon la revendication 4, comprenant en outre l'étape consistant à conditionner l'acide phénolsulfonique convenant pour traiter une ou plusieurs affections parmi la stomatite aphteuse, la parodontite chronique, et la gingivite.

6. Procédé selon la revendication 4, comprenant en outre l'étape consistant à conditionner les sels sulfonates convenant pour traiter une ou plusieurs affections dermatologiques et pour le resurfaçage cutané ou les aphtes.

7. Produit fabriqué par le procédé selon la revendication 1.

8. Produit fabriqué par le procédé selon la revendication 3.

9. Formulation utilisable dans le traitement des discontinuités des muqueuses, comprenant: des isomères de l'acide phénolsulfonique, des isomères de l'acide gaïacolsulfonique, et éventuellement, de l'acide libre et de l'eau libre, la formulation pouvant être obtenue par le procédé selon la revendication 1.

10. Formulation utilisable dans le traitement des discontinuités des muqueuses, comprenant: de l'acide phénolsulfonique et des isomères de l'acide phénolsulfonique; de l'acide gaïacolsulfonique et des isomères de l'acide gaïacolsulfonique et des formes mono et bis de l'acide gaïacolsulfonique; du phénolsulfonate d'ammonium; et du gaïacolsulfonate de potassium, les isomères de l'acide phénolsulfonique et de l'acide gaïacolsulfonique pouvant être obtenus par le procédé selon la revendication 1.

11. Formulation utilisable dans le traitement des discontinuités des muqueuses orales, comprenant: de l'acide phénolsulfonique à une concentration de 25-80% en poids; de l'acide gaïacolsulfonique à une concentration de 25-80% en poids; de l'acide sulfurique libre à une concentration de 0 à 32% en poids; et de l'eau à une concentration de 0 à 3% en poids.

12. Formulation utilisable dans le traitement des discontinuités des muqueuses cutanées, comprenant: de l'acide phénolsulfonique à une concentration de 25-80% en poids; du phénolsulfonate d'ammonium à une concentration de 0 à 5% en poids; de l'acide gaïacolsulfonique à une concentration de 25-80% en poids; de l'acide sulfurique libre à une concentration de 0 à 32% en poids; et de l'eau à une concentration de 0 à 3% en poids.

13. Trousse utilisable dans le traitement des discontonuités des muqueuses comprenant la formulation selon la revendication 10, 11 ou 12, un récipient destiné à contenir la formulation et un mécanisme destiné à administrer la formulation à une discontinuité de muqueuse.

14. Dispositif utilisable dans le traitement des discontinuités des muqueuses, comprenant:
une seringue, et
le produit selon la revendication 7 contenu dans la seringue.

15. Système comprenant:
des ingrédients qui incluent de l'acide phénolsulfonique, de l'acide gaïacolsulfonique, du phénolsulfonate d'ammonium, du gaïacolsulfonate de potassium, de l'eau et de l'acide libre; et
un mécanisme réglé pour mélanger des quantités prédéfinies d'au moins deux des ingrédients ensemble pour préparer une formulation utilisable dans le traitement d'un type spécifique de discontinuité de muqueuse.

16. Système selon la revendication 15, comprenant en outre un ou plusieurs composés parmi l'acide salicylique et l'acide sulfosalicylique.

17. Système selon la revendication 15, dans lequel l'acide phénolsulfonique, l'acide gaïacolsulfonique, le phénolsulfonate d'ammonium et le gaïacolsulfonate de potassium sont mélangés de manière dosée pour préparer une formulation adaptée au traitement de la gingivite, ladite formulation ne contenant que peu ou pas du tout d'acide libre.

18. Système selon la revendication 15, dans lequel les acides phénolsulfoniques et l'acide gaïacolsulfonique sont mélangés de manière dosée pour former une formulation contenant de l'acide sulfurique libre, ladite formulation étant utile pour traiter une parodontopathie.

19. Système selon la revendication 15, dans lequel l'acide phénolsulfonique et l'acide gaïacolsulfonique sont mélangés de manière dosée à des concentrations efficaces pour servir d'exfoliant facial ou de traitement des aphtes.

20. Système selon la revendication 15, dans lequel l'acide phénolsulfonique, le phénolsulfonate, et l'acide libre sont mélangés de manière dosée à des concentrations efficaces pour servir d'exfoliant pour les pieds.

21. Composition exfoliante, comprenant: un mélange comprenant de l'acide phénolsulfonique, de l'acide gaïacolsulfonique et, éventuellement, de l'acide sulfosalicylique.

22. Composition exfoliante selon la revendication 21, comprenant en outre un ou plusieurs composants parmi un agent émollient, un agent hydratant, un agent épaississant, et un agent émulsifiant, un agent neutralisant, un agent colorant, et un absorbeur ou filtre UV, et un conservateur.

23. Composition exfoliante selon la revendication 21, dans laquelle le mélange est un gel.

24. Composition exfoliante selon la revendication 21, dans laquelle le mélange est une crème ou une pâte ou une mousse.

25. Composition exfoliante selon la revendication 21, dans laquelle le mélange a un pH compris entre 2 et 6.

26. Composition exfoliante selon la revendication 21, dans laquelle l'acide phénolsulfonique a une concentration pouvant atteindre au moins 24% en volume du mélange.

27. Composition exfoliante selon la revendication 21, dans laquelle la concentration d'acide phénolsulfonique est de 4% en volume du mélange.

28. Composition exfoliante selon la revendication 21, comprenant en outre de l'acide citrique.
